# FASCICULE DE BREVET EUROPEEN

(11) **EP 4 015 034 B1**
(45) Date de publication et mention de la délivrance du brevet: **14.12.2022**
(21) Numéro de dépôt: 20315507.2
(22) Date de dépôt: 28.12.2020
(51) Int. Cl.: A61N 1/372, A61N 1/375, A61N 1/378

(54) **IMPLANT CARDIAQUE AUTONOME DE TYPE CAPSULE LEADLESS, COMPRENANT UNE INTERFACE DE RECHARGE DE BATTERIE ET DE COMMUNICATION EXTERNE PENDANT LE TRANSPORT ET LE STOCKAGE**
AUTONOMES HERZIMPLANTAT VOM TYP LEITUNGSLOSE KAPSEL MIT EINER SCHNITTSTELLE ZUM AUFLADEN DER BATTERIE UND ZUR EXTERNEN KOMMUNIKATION WÄHREND DES TRANSPORTS UND DER LAGERUNG
LEADLESS CAPSULE AUTONOMOUS CARDIAC IMPLANT, COMPRISING AN INTERFACE FOR BATTERY RECHARGING AND EXTERNAL COMMUNICATION DURING TRANSPORT AND STORAGE

(43) Date de publication de la demande: 22.06.2022
(60) Demande divisionnaire: 21185223.1
(73) Titulaire: Cairdac, 92160 Antony (FR)
(72) Inventeur: Makdissi, Alaa, 75011 Paris (FR); Regnier, Willy, 91160 Longjumeau (FR); Nguyen-Dinh, An, 37520 La Riche (FR)
(74) Mandataire: Dupuis-Latour, Dominique

(56) Documents cités:
- EP-A1- 2 857 065
- EP-A1- 3 693 056
- EP-A1- 3 708 221
- WO-A1-98/08567
- US-A1- 2019 091 479

## Description

### CONTEXTE DE L'INVENTION

### Domaine technique

L'invention concerne les dispositifs médicaux durant leur transport et leur stockage, pendant la période comprise entre leur fabrication et le moment où ils sont utilisés par le praticien afin d'être implantés chez un patient.

Elle concerne plus précisément ceux de ces dispositifs qui incorporent un système d'auto-alimentation comprenant un récupérateur d'énergie mécanique, également dénommé "harvester" ou "scavenger" associé à un organe de stockage d'énergie intégré tel qu'une micro-batterie tampon rechargeable ou une capacité à hautes performances.

Le récupérateur peut être notamment du type dit "PEH" (*Piezoelectric Energy Harvester*), qui utilise comme transducteur mécanoélectrique une lame piézoélectrique oscillante couplée à une masse mobile inertielle.

Le transport et le stockage de tels dispositifs implique, comme on l'expliquera plus bas, un certain nombre de contraintes et difficultés spécifiques, propres à la présence du harvester, et qui ne se rencontrent pas avec les dispositifs médicaux conventionnels alimentés par une pile statique de très longue durée de vie (dispositifs tels que stimulateurs cardiaques, défibrillateurs implantables, etc.).

### État de la technique antérieure

Les récupérateurs de type "harvester" sont notamment utilisés pour alimenter des dispositifs médicaux autonomes implantables (ci-après "implants"), en particulier des capsules autonomes destinées à être implantées dans une cavité cardiaque.

La description n'est toutefois pas limitée à un tel dispositif, elle est applicable aussi bien à de nombreux autres types de dispositifs médicaux implantables miniaturisés, quelle que soit leur destination fonctionnelle, cardiaque ou autre.

L'un des aspects critiques de ces dispositifs miniaturisés est celui de l'autonomie électrique. En effet, la durée de vie d'un tel dispositif étant d'environ 8 à 10 ans, compte tenu des très faibles dimensions il n'est pas possible d'utiliser une batterie conventionnelle, même à forte densité. Un harvester pallie cet inconvénient, en recueillant l'énergie mécanique résultant des mouvements divers subis par le corps de l'implant. Ces mouvements peuvent avoir pour origine un certain nombre de phénomènes se produisant notamment au rythme des battements cardiaques, tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie est convertie au moyen d'un transducteur mécanoélectrique approprié en une énergie électrique (tension ou courant) suffisante pour assurer l'alimentation des divers circuits et capteurs du dispositif et la recharge de l'organe de stockage d'énergie. Ce système d'alimentation permet ainsi au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules leadless", pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

Dans le cas d'une application cardiaque, la capsule leadless surveille en continu le rythme du patient et délivre si nécessaire au cœur des impulsions électriques de stimulation, de resynchronisation et/ou de défibrillation en cas de trouble du rythme détecté par la capsule. La capsule leadless peut être une capsule épicardique, fixée à la paroi extérieure du cœur, ou bien une capsule endocavitaire, fixée à la paroi intérieure d'une cavité ventriculaire ou auriculaire, ou bien encore une capsule fixée sur une paroi d'un vaisseau à proximité du myocarde. La fixation de la capsule au site d'implantation est assurée par un système d'ancrage saillant prolongeant le corps de la capsule et destiné à pénétrer dans le tissu cardiaque, notamment au moyen d'une vis.

La capsule comprend divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Les US 2009/0171408 A1 (Solem), US 2017/0151429 A1 (Regnier) et WO 2018/122244 A1 (Regnier) décrivent divers exemples de telles capsules leadless intracardiaques.

Le récupérateur d'énergie intégré à ces capsules peut notamment mettre en œuvre un ensemble pendulaire inertiel soumis aux sollicitations externes décrites plus haut. Une masse mobile (dite "masse sismique") couplée à un élément élastiquement déformable est entrainée au gré des mouvements de la capsule et vibre à une fréquence propre d'oscillation libre. L'énergie mécanique de l'oscillation est convertie en énergie électrique par un transducteur mécanoélectrique, qui dans le cas d'un PEH est un composant piézoélectrique tel qu'une lame encastrée à l'une de ses extrémités et couplée à la masse inertielle à son autre extrémité, qui est libre. La lame, sollicitée de façon cyclique et alternative en flexion, engendre des charges électriques qui sont récupérées en surface du composant pour être utilisées par le système d'autoalimentation de la capsule afin d'assurer l'alimentation des divers circuits électroniques et capteurs, ainsi que la recharge de la micro-batterie tampon.

Un tel récupérateur d'énergie de type PEH destiné à l'alimentation d'un implant à partir des oscillations d'une lame piézoélectrique est notamment décrit dans le US 3,456,134 A (Ko) et dans les WO 2019/001829 A1 (Cairdac) et EP 3 708 221 A1 (Cairdac).

Dans la phase préalable à l'implantation, c'est-à-dire pendant le transport et le stockage de l'implant, il est toutefois nécessaire de maintenir pendant toute la durée du stockage (qui peut durer de nombreux mois) un niveau de charge de la micro-batterie tampon intégrée qui soit suffisant pour maintenir en veille les circuits électriques de l'implant jusqu'au moment de l'implantation, moment auquel ces circuits seront activés pour devenir pleinement fonctionnels.

La consommation de l'implant en veille est certes très faible, de l'ordre de 100 nA à 1 µA, incluant l'alimentation des circuits de veille et le courant d'autodécharge de l'organe de stockage d'énergie. Mais les organes de stockage d'énergie utilisés avec les implants miniaturisés, qu'il s'agisse de micro-batteries tampon rechargeables ou de condensateurs de forte capacité (dans la suite on utilisera le terme générique de "batterie") ont une capacité limitée, typiquement à une valeur de l'ordre de 1 mAh à 10 mAh, ce qui assure une autonomie sur étagère d'environ 1000 h dans le pire des cas, soit approximativement 40 jours seulement - en effet, sur étagère l'implant est immobile et il n'y a donc pas de recharge par le harvester.

Ces chiffres sont à comparer à ceux des implants traditionnels (pacemakers, défibrillateurs implantables, etc.) munis d'une pile statique de longue durée, dont la capacité est généralement d'au moins 100 mAh : dans ce cas, pour un même courant de veille de 1 µA, au bout de 20 mois de stockage la pile n'aura perdu que 15 % de sa capacité nominale.

La demande EP 20315501.5, déposée le 22.12.2020 au nom du Demandeur pour un "*Accessoire pour le transport et le stockage d'un implant cardiaque autonome de type capsule leadless*", décrit un moyen permettant de garantir l'alimentation des circuits de veille de l'implant dans une état où celui-ci est immobile donc sans fonctionnement du harvester (stockage sur étagère avant implantation), et ce pendant une très longue durée, typiquement d'au moins 24 mois. Essentiellement, la capsule est couplée par voie galvanique à une source externe d'énergie électrique matériellement séparée de l'implant, par exemple une pile logée dans le même emballage que celui où est conditionné l'implant. La source externe est couplée à la batterie rechargeable de l'implant de manière à assurer une alimentation de cette batterie rechargeable par la source externe et maintenir ainsi, pendant toute la durée du transport et du stockage avant implantation, un niveau de charge de la batterie supérieur à un niveau minimal prédéterminé.

Une configuration comparable est décrite par le WO 98/08567 A1 (Pacesetter), où l'implant est couplé à une source externe d'énergie permettant d'alimenter l'implant pendant toute la durée du stockage, préservant ainsi la pile interne d'alimentation de ce dernier. Un circuit d'interfaçage permet de commuter à volonté l'alimentation des circuits de l'implant soit sur la source externe, soit sur la pile interne.

Toutefois, dans cette configuration, un *premier problème* se présente, à savoir la manière d'assurer, hors de la capsule, le couplage galvanique de la source externe à la capsule et, à l'intérieur de la capsule, jusqu'à la batterie rechargeable.

Il s'agit en particulier de garantir qu'une fois que la capsule aura été implantée et sera pleinement fonctionnelle pour assurer les fonctions requises notamment de détection/stimulation de potentiel cardiaque, les moyens mis auparavant en oeuvre pour maintenir le niveau de charge de la batterie lors du stockage n'auront aucune incidence sur le fonctionnement de l'implant, et ne créeront aucun risque de dysfonctionnement matériel ni logiciel.

Un *deuxième problème* réside dans la multiplication des électrodes externes de l'implant du fait de la fonction de recharge de la batterie en configuration de stockage. Le couplage galvanique de la source externe d'énergie électrique (la pile logée dans l'emballage de l'implant) à la batterie rechargeable à l'intérieur de l'implant nécessite en effet d'établir un contact physique avec des électrodes présentes en surface du corps tubulaire de l'implant.

Il est certes envisageable d'ajouter sur le corps tubulaire des électrodes (surfaces conductrices) exclusivement dédiées à la fonction de recharge de la batterie, mais cette solution complique la conception mécanique de l'implant, notamment de son corps tubulaire constitué d'un assemblage soudé de pièces conductrices en métal (généralement en titane) et de pièces électriques électriquement isolantes (généralement en céramique). Une augmentation du nombre d'électrodes se traduirait alors par une multiplication du nombre de pièces à assembler et du nombre de soudures, avec par voie de conséquence un accroissement de la difficulté de réalisation et une augmentation du coût de fabrication.

Par ailleurs, un *troisième problème* résulte de la nécessité de maintenir, même pendant le stockage, la possibilité d'échanger des données entre, d'une part, la capsule dans son état dormant et, d'autre part le monde extérieur : en effet, avant toute implantation, il est nécessaire de pouvoir entrer en communication avec la capsule pour la "réveiller" de son état dormant, vérifier le niveau de charge de la batterie interne, tester la capsule avant de l'implanter pour s'assurer de l'absence d'anomalie fonctionnelle, ajuster le paramétrage des circuits de détection/stimulation pour les adapter à l'état clinique du patient, etc.

À ce sujet, le US 2019/070422 A1 (Regnier) décrit une capsule leadless pourvue de moyens de communication avant implantation, c'est-à-dire dans une situation où elle est encore configurée pour le stockage et le transport. L'échange de données entre la capsule et l'environnement extérieur repose sur une technique de communication par voie intracorporelle dite HBC (*Human Body Communication*), qui est une technique où la communication est conduite par un milieu constitué par les tissus ou fluides interstitiels du corps d'un patient, et ne peut de ce fait être normalement mis en œuvre qu'après implantation. Le document propose d'adjoindre à la capsule un accessoire permettant néanmoins d'utiliser un couplage capacitif (galvaniquement isolé) entre la capsule et un boitier externe pour échanger des données par cette voie alors que la capsule n'est pas encore implantée.

Une autre technique de communication consiste à échanger des données par transmission RF sans fil (signaux de télémétrie Bluetooth BLE ou dans les bandes ISM).

Mais dans l'un ou l'autre cas la capsule est supposée être déjà dans un état pleinement fonctionnel, et non dans un état dormant nécessité par un stockage prolongé.

### RÉSUMÉ DE L'INVENTION

Pour résoudre les problèmes ci-dessus, l'invention propose un implant cardiaque autonome notamment de type capsule leadless, du type connu décrit par le EP 3 708 221 A1 précité, comprenant les caractéristiques visées au préambule de la revendication 1.

L'implant selon l'invention comprend, de façon caractéristique, les éléments constitutifs énoncés à la partie caractérisante de la revendication 1.

Les sous-revendications visent diverses formes de réalisation subsidiaires, avantageuses, de l'invention.

### DESCRIPTION SOMMAIRE DES DESSINS

La Figure 1 illustre une capsule leadless dans son environnement, implantée au fond du ventricule droit du myocarde d'un patient.
La Figure 2 est une vue longitudinale générale d'une capsule *leadless* comprenant un récupérateur d'énergie à ensemble pendulaire.
La Figure 3 présente sous forme schématique les principaux blocs fonctionnels constitutifs d'une capsule leadless.
La Figure 4 est une vue générale illustrant le conditionnement complet, avec l'implant et ses accessoires enfermés dans un emballage étanche stérile.
La Figure 5 est une vue en élévation, partiellement en coupe, de l'implant placé sur son support de protection et de calage, en situation pendant le transport et le stockage.
La Figure 6 est un schéma électrique expliquant la manière dont fonctionne le système de recharge de la batterie tampon de l'implant.
La Figure 7 illustre un exemple de circuiterie permettant d'assurer à l'intérieur de la capsule un interfaçage entre les électrodes de surface et la batterie tampon pour le couplage temporaire de cette dernière à une pile externe, dans le cas où la capsule est du type à stimulation par envoi d'impulsions négatives sur une électrode d'extrémité.
La Figure 8 illustre une variante de la circuiterie de la Figure 7, destinée à une capsule du type à stimulation par envoi d'impulsions positives sur une électrode annulaire.
La Figure 9 est un schéma électrique fonctionnel expliquant la manière d'établir une communication bidirectionnelle entre la capsule et le monde extérieur avec le couplage galvanique destiné à la recharge de la batterie tampon par une pile externe.
La Figure 10 illustre des exemples de signaux échangés avec la configuration électrique de la Figure 9.

### DESCRIPTION DÉTAILLÉE DE MODES DE RÉALISATION PRÉFÉRENTIELS DE L'INVENTION

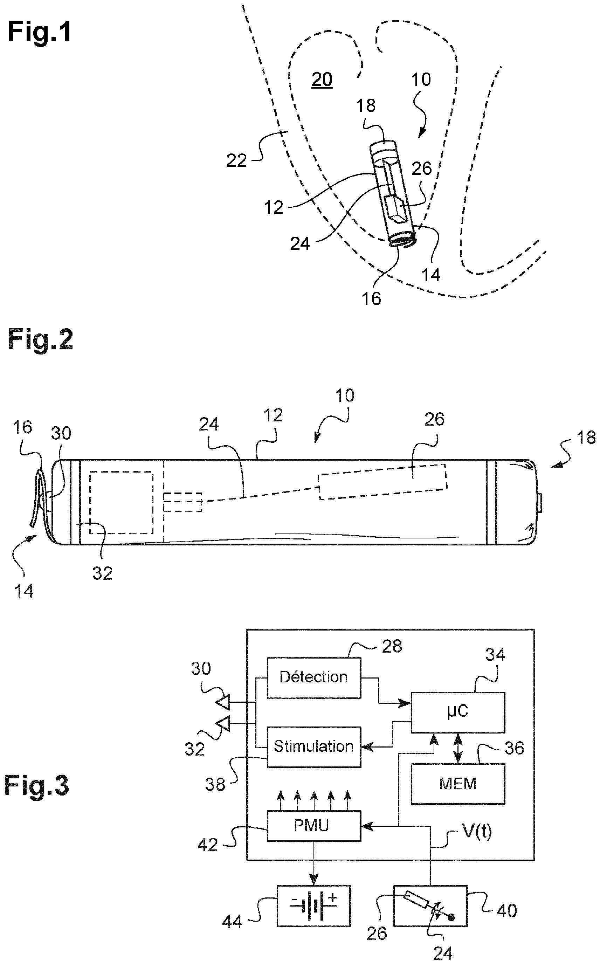

Sur les Figures 1 et 2 on a représenté un implant de type capsule leadless 10 dans une application à la stimulation cardiaque.

La capsule 10 se présente extérieurement sous la forme d'un corps tubulaire allongé cylindrique 12 enfermant les divers circuits électroniques et d'alimentation de la capsule ainsi qu'un récupérateur d'énergie à ensemble pendulaire. Les dimensions typiques d'une telle capsule sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. L'extrémité opposée (proximale) 18 de la capsule 10 est une extrémité libre, qui est seulement pourvue de moyen de liaison temporaire à un cathéter-guide (non représenté) ou autre accessoire d'implantation pour la mise en place ou l'explantation de la capsule.

Dans l'exemple illustré Figure 1, la capsule leadless 10 est un implant endocavitaire implanté à l'intérieur d'une cavité 20 du myocarde 22, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde.

La capsule leadless 10 est dotée d'un module récupérateur d'énergie comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques. L'ensemble pendulaire peut en particulier être constitué d'une lame piézoélectrique 24 encastrée à l'une de ses extrémités et dont l'extrémité opposée, libre, est couplée à une masse inertielle mobile 26, l'ensemble formant un système pendulaire de type masse-ressort. Du fait de son inertie, la masse 26 soumet la lame 24 à une déformation de type vibratoire de part et d'autre d'une position neutre ou non déformée correspondant à une position stable de repos en l'absence de toute sollicitation. La lame piézoélectrique 24 assure en outre une fonction de transducteur mécanoélectrique permettant de convertir la contrainte mécanique de flexion qui lui est appliquée en charges électriques qui sont collectées pour produire un signal électrique qui, après redressement, stabilisation et filtrage alimentera les divers circuits électroniques de la capsule.

La Figure 3 est un synoptique des divers circuits électriques et électroniques intégrés à la capsule leadless, présentés sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule (voir Figure 2). Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer en tant que de besoin au système d'électrodes 30, 32 des impulsions de stimulation du myocarde.

Il est par ailleurs prévu un circuit récupérateur d'énergie PEH 40, constitué par l'ensemble pendulaire formé par la lame piézoélectrique 24 et la masse inertielle 30 décrits plus haut aux Figures 1 et 2. La lame piézoélectrique 24 assure aussi une fonction de transducteur mécanoélectrique qui convertit en charges électriques les sollicitations mécaniques subies et produit un signal électrique variable V(t), qui est un signal alternatif oscillant à la fréquence d'oscillation libre de l'ensemble pendulaire lame 24/masse 30 et au rythme des battements successifs du myocarde auquel la capsule est couplée.

Le signal électrique variable V(t) est délivré à un circuit de gestion de l'énergie ou PMU 42. Le PMU 42 redresse et régule le signal V(t) de manière à produire en sortie une tension ou un courant continus stabilisés servant à l'alimentation des divers circuits électroniques et à la recharge d'une micro-batterie tampon intégrée 44 (on assimilera au cas d'une micro-batterie celui d'un condensateur de forte capacité, qui remplit la même fonction de stockage temporaire d'une énergie électrique permettant d'assurer l'alimentation de l'ensemble des circuits de l'implant).

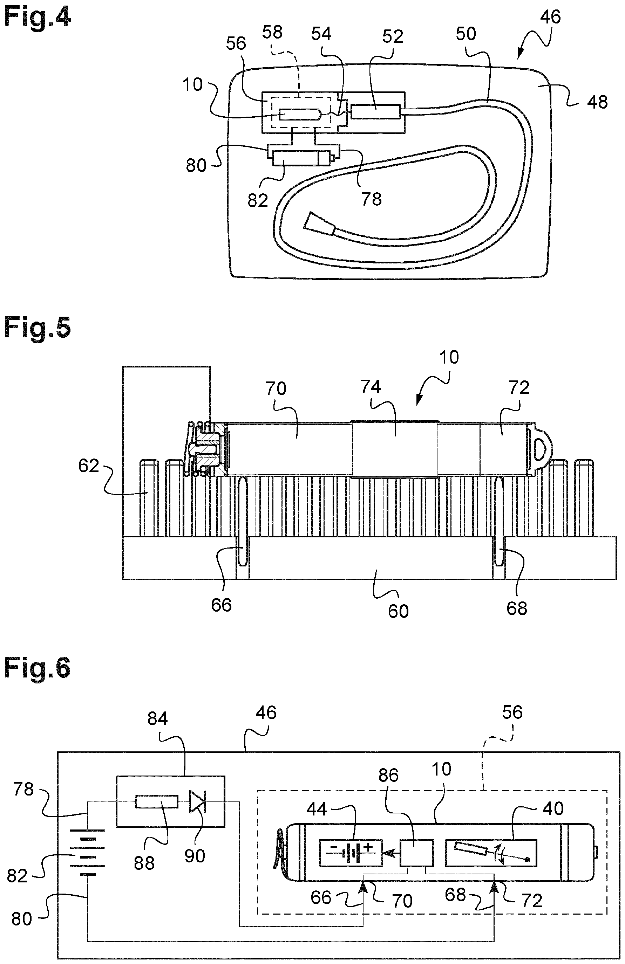

La Figure 4 est une vue générale illustrant le conditionnement complet, avec l'implant et ses accessoires enfermés dans un emballage étanche stérile.

Le conditionnement comprend un emballage étanche 46 définissant un volume interne étanche et stérile 48 dans lequel est enfermée la capsule 10. L'emballage contient également, outre la capsule, un cathéter 50 destiné à l'implantation, qui se termine côté distal (à proximité de la capsule) par un logement ou "housing" 52 recevant et protégeant la capsule pendant le guidage dans le réseau veineux et empêchant également que la vis d'ancrage 16 ne vienne blesser les parois vasculaires. Dans l'emballage, la capsule est sortie du housing 52 et n'est reliée au cathéter que par un fil de sécurité ou "fil d'Ariane" 54 dont elle ne sera libérée qu'une fois atteint le site d'implantation définitif.

La capsule 10 est disposée à l'intérieur d'un support de protection et de calage 56 incluant une structure d'amortissement 58 comprenant par exemple, comme illustré Figure 5, une pièce 60 supportant une texture de brins ou lamelles souples déformables 62 ou, en variante, un bloc massif en mousse.

Outre la protection mécanique, il est prévu d'établir un couplage électrique avec des électrodes de la capsule, de manière à pouvoir recharger en tant que de besoin la batterie tampon intégrée à la capsule, de la manière qui sera exposée plus bas en référence à la Figure 6.

Pour cela, il est prévu des pointes de touche 66, 68 venant en contact avec des surfaces conductrices distinctes 70, 72 du corps tubulaire 12 de la capsule 10. Une telle structure de corps tubulaire comprenant deux surfaces conductrices 70, 72 (métalliques) séparées par une surface cylindrique isolante 74 (céramique) est décrite par exemple dans le EP 3 730 185 A1 (Cairdac), auquel on pourra se référer pour plus de détails.

Les pointes de touche 66, 68 peuvent être des tiges avec une extrémité télescopique ou une bille rétractable venant en contact avec les surfaces conductrices 70, 72 ; en variante, le couplage électrique peut être réalisé par des lames souples ou des ressorts conducteurs, ou par tout autre moyen assurant la même fonction.

Les pointes de touche 66, 68 sont reliées par des conducteurs respectifs 78, 80 à une source d'énergie électrique 82 (Figure 4), déportée par rapport au support 56 de protection et de calage de la capsule. La source d'énergie électrique peut être une pile conventionnelle, par exemple de 1,5 V. En variante, la source d'énergie électrique déportée peut être un récepteur d'énergie inductive, par exemple une boucle de recharge par induction disposée dans le volume interne 48 du conditionnement stérile de l'emballage 46 ; cette boucle est alors couplée à un émetteur d'énergie inductive situé à l'extérieur du conditionnement stérile 46.

On va maintenant décrire, en référence au schéma électrique de la Figure 6, la manière dont le niveau de charge de la batterie tampon 44 peut être maintenu à un niveau minimal satisfaisant malgré l'absence de recharge par le harvester 40. Les pointes de touche 66, 68 assurent un couplage galvanique de la capsule 10 à la source d'énergie électrique déportée 82 (ci-après désignée "pile" par souci de simplicité).

La tension nominale de la pile 82 est choisie de manière à être supérieure à la tension opérationnelle de la batterie tampon 44, par exemple une tension de pile de 1,5 à 9 V, typiquement de 5 à 6 V, pour une tension de batterie tampon variant typiquement entre 3 V et 4,2 V. Si la tension de la pile est inférieure à celle de la batterie tampon, on peut prévoir un circuit *boost* élévateur de tension, soit externe à la capsule, soit interne à celle-ci (par exemple un étage élévateur de tension au sein du PMU 42 (Figure 3)).

Le couplage de la pile 82 à la batterie tampon 44 comprend, outre les pointes de touche 64, 66, un circuit 84 d'interfaçage entre la pile 82 et la capsule 10, et un circuit d'interfaçage 86 interne à la capsule permettant de coupler les surfaces externes conductrices 70, 72 à la batterie tampon 44.

Dans sa configuration la plus simple, le circuit 84 d'interfaçage pile/capsule comprend une résistance 88 de limitation du courant de recharge délivré par la pile, et une diode 90 permettant d'interrompre la recharge lorsque le niveau de tension de la batterie 44 atteint la valeur de la tension de la pile 82. Dans une variante plus élaborée, le circuit d'interfaçage 84 peut comprendre un circuit permettant de déterminer le niveau de tension de la batterie et de commander sélectivement la délivrance du courant de recharge, en interrompant l'alimentation de la batterie 44 par la pile 82 lorsque le niveau de charge dépasse un seuil haut prédéfini, et en rétablissant cette alimentation lorsque le niveau de charge a chuté jusqu'à un seuil bas prédéfini.

Par ailleurs, il peut être prévu un moyen de contrôle visuel, par exemple au moyen d'une LED (non représentée), du couplage correct entre la pile 82 et la capsule 10, c'est-à-dire de vérification du bon état de la fonctionnalité de recharge contrôlée de la batterie tampon de la capsule par la pile 82 à l'intérieur du conditionnement étanche.

Du point de vue quantitatif, pour un courant de veille et une autodécharge de la batterie entraînant un courant permanent de l'ordre de 1 µA et pour une capacité de la batterie de l'ordre de 1 mAh, on aboutit normalement à une longévité sur étagère de l'ordre de 1000 h, soit environ 40 jours, du fait de l'absence de recharge par le harvester, qui est immobile.

Pour garantir une durée de stockage de 24 mois pendant laquelle on veut être certain que la capsule reste fonctionnelle bien qu'en veille, il sera nécessaire de prévoir environ 30 cycles de recharge de la batterie 44 par la pile 82. Si l'on estime le rendement de l'opération à 50 %, ceci donne pour la pile externe 82 une capacité de 60 mAh, valeur tout à fait compatible avec ce que procurent les piles "bouton" conventionnelles, qui ont typiquement une capacité de l'ordre de 80 à 100 mAh ou plus.

Il est ainsi possible, avec des moyens très simples, de garantir en toutes circonstances un stockage sur étagère de très longue durée, sans diminution aucune de la longévité de la capsule, celle-ci étant toujours fonctionnelle et prête à être réveillée à tout moment pour permettre son implantation.

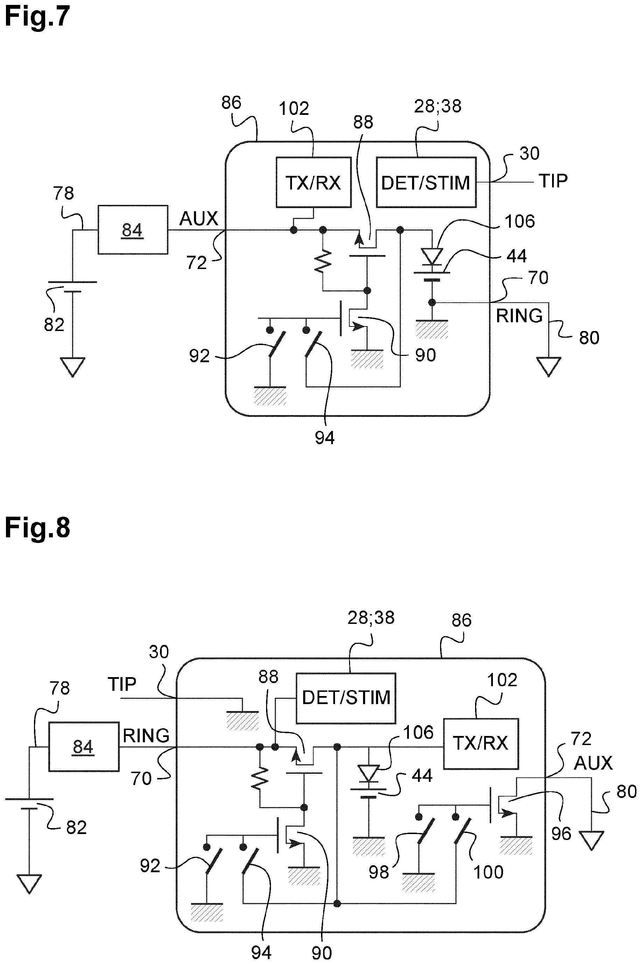

La Figure 7 illustre un exemple de circuiterie permettant d'assurer l'interfaçage entre les électrodes de surface de la capsule et la pile externe 82 via le couplage galvanique comprenant les conducteurs de liaison 78, 80.

Dans cet exemple, l'une des électrodes est avantageusement (mais de façon non limitative) une électrode annulaire préexistante de la capsule, faisant fonction d'électrode d'anode pour la détection/stimulation. Cette électrode est par exemple l'électrode annulaire 32 illustrée Figure 2, ou l'électrode annulaire 70 illustrée Figure 5. Elle sera désignée par la suite électrode RING. L'autre électrode de stimulation, ci-après désignée électrode TIP, est une électrode d'extrémité telle que l'électrode de cathode 30 illustrée Figure 2. Cette électrode distale est habituellement recouverte d'un revêtement de type nitrure de titane de très faible épaisseur, très performant sur le plan de la stimulation mais très fragile et sensible aux rayures et contaminations particulaires ; il est de ce fait préférable de la réserver à la seule fonction de stimulation en évitant tout contact par exemple avec une pointe de touche comme dans cas de l'électrode annulaire 70 (point de touche 66 sur la Figure 5).

La capsule est pourvue d'une électrode de surface additionnelle, ci-après désignée électrode AUX, par exemple une électrode 72 illustrée Figure 5 en contact avec la pointe de touche 68, électriquement isolée de l'électrode RING 70 par la partie tubulaire électriquement isolante 74 en céramique.

Dans la configuration de stockage, l'électrode de surface annulaire AUX 72 est reliée au pôle positif de la pile externe 82 par la pointe de touche 68 et le conducteur 78, et l'électrode annulaire RING 70 est reliée au pôle négatif de la pile externe 82 par la pointe de touche 68 et le conducteur 80.

L'électrode AUX sert seulement à assurer la fonction de recharge de la batterie décrite plus haut en référence à la Figure 6 ; en revanche, l'électrode RING a une double fonction : pour le couplage à la pile externe 82 afin de recharger la batterie dans la phase de transport et de stockage, et comme électrode de stimulation (électrode d'anode) lorsque la capsule est pleinement fonctionnelle, après implantation.

Il est donc nécessaire de commuter cette électrode RING de manière à lui faire assurer ce double rôle, au moyen de circuits qui vont maintenant être décrits plus en détail.

Dans la configuration illustrée Figure 7, le mode de stimulation utilisé est celui le plus couramment rencontré en pratique, par émission d'impulsions négatives appliquées sur l'électrode TIP. Pour cela, l'électrode TIP est directement reliée aux circuits 28, 38 de détection/stimulation, et l'électrode RING est reliée à la masse des circuits de la capsule.

Le couplage temporaire à la pile externe 82 est assuré pendant le stockage par un transistor PMOS 88 reliant l'électrode AUX à la batterie tampon 44. La grille de ce PMOS 88 est commandée par un NMOS 90 dont la grille est reliée à deux interrupteurs parallèles 92, 94 eux-mêmes reliés, respectivement, à la masse des circuits de la capsule et au pôle positif de la batterie tampon 44. Dans la configuration de transport et de stockage l'interrupteur 92 est ouvert et l'interrupteur 94 est fermé : la grille du NMOS 90 étant mise à un potentiel positif, le NMOS 90 rend passant le PMOS 88, reliant ainsi le pôle positif de la batterie tampon 44 à l'électrode de surface AUX et, par voie de conséquence, à la pile externe 82 par le couplage 78. La fonction de recharge de la batterie tampon interne 44 par la pile externe 82 peut être ainsi assurée.

Après implantation, la position des interrupteurs 92 et 94 est inversée, avec l'interrupteur 92 fermé et l'interrupteur 94 ouvert. La grille du NMOS 90, mise à la masse, bloque le PMOS 88 et déconnecte de ce fait la batterie tampon 44 de l'électrode AUX. Les deux transistors MOS 88 et 90 étant bloqués, l'électrode AUX devient flottante et ainsi ne risque en aucune façon de perturber la détection et la stimulation par les circuits 28, 38 et les électrodes TIP et RING. Très avantageusement, les interrupteurs 92 et 94 sont des interrupteurs de type OTP (*One Time Programmable*), qui sont donc des composants fusibles qui conservent leur état fermé ou ouvert quelles que soient les circonstances, notamment quelle que soit la tension de la batterie tampon 44 en cours de fonctionnement. Surtout, avec de tels composants, la transition de la configuration de transport et de stockage à la configuration fonctionnelle finale sera une transition irréversible, garantissant que l'électrode AUX demeure à l'état flottant pendant tout le reste de la durée de vie de la capsule.

La Figure 8 illustre une variante de la Figure 7, adaptée aux cas d'un implant avec lequel la stimulation se fait par envoi d'impulsions positives sur l'électrode annulaire RING (et non par envoi d'impulsions négatives sur l'électrode distale TIP).

Dans ce cas, l'électrode TIP est reliée de façon permanente à la masse interne des circuits de l'implant, tandis que l'électrode RING est reliée aux circuits de détection/stimulation 28, 38. Pour pouvoir contrôler les deux électrodes RING et AUX, il est prévu un transistor NMOS supplémentaire 96 dont la grille est pilotée par des interrupteurs supplémentaires 98, 100 dont le fonctionnement est le même que celui des interrupteurs 92 et 94 décrits précédemment en référence à la Figure 7 : pendant la phase de transport et de stockage, les interrupteurs 92 et 98 sont ouverts et les interrupteurs 94 et 100 sont fermés, tandis qu'après implantation les interrupteurs 92 et 98 sont fermés et les interrupteurs 94 et 100 sont ouverts, rendant ainsi totalement et définitivement flottante l'électrode AUX. Les interrupteurs 98 et 100 sont avantageusement, comme les interrupteurs 92 et 94, des composants OTP.

On va maintenant décrire la manière dont, très avantageusement, il est possible d'utiliser le couplage galvanique existant entre la capsule et la pile externe non seulement pour assurer la recharge de la batterie tampon interne, mais également pour réaliser une communication filaire bidirectionnelle entre la capsule et le monde extérieur.

Comme illustré Figures 7 et 8, la capsule comprend un circuit interne émetteur/récepteur TX/RX 102, qui est relié par l'intermédiaire de la diode 106 au pôle positif de la batterie tampon 44.

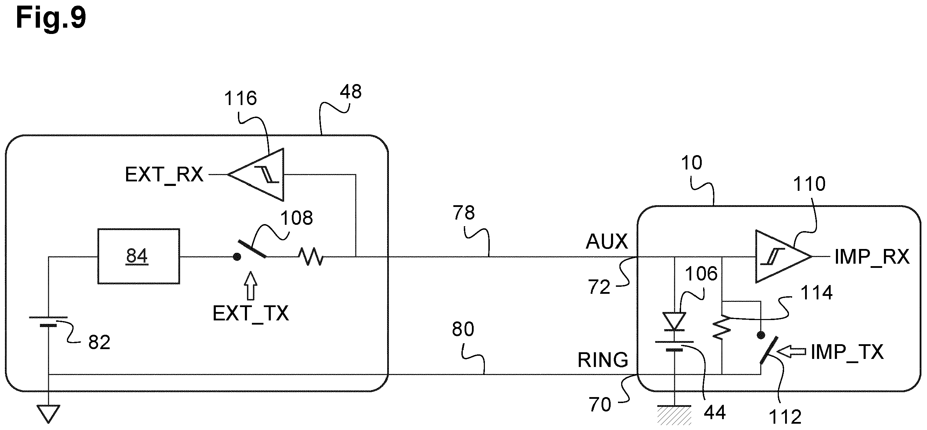

Le schéma équivalent est celui illustré Figure 9 avec, à droite, l'implant 10 et, à gauche, son environnement extérieur, par exemple l'environnement du volume étanche 48 du conditionnement de transport et de stockage 46.

Le principe de base de la communication entre la capsule et l'environnement extérieur consiste à échanger de brèves impulsions de tension :
- dans le sens allant de l'environnement extérieur vers la capsule (la capsule fonctionnant alors en réception), par de brèves interruptions de la tension de recharge appliquée à la batterie tampon, et
- dans le sens allant de la capsule vers l'environnement extérieur (la capsule fonctionnant alors en émission), par de brèves modifications de l'impédance de charge de la capsule vue depuis l'extérieur.

Du côté de l'environnement extérieur, un commutateur 108 interposé entre la pile externe 82 et la ligne d'alimentation 78 de l'implant est piloté par un signal de commande EXT_TX. Les fermetures/ouvertures résultantes du commutateur 108 produisent de très brèves coupures de la tension d'alimentation délivrée à la capsule, qui du fait de leur brièveté seront sans incidence sur la fonction de recharge de la batterie tampon 44.

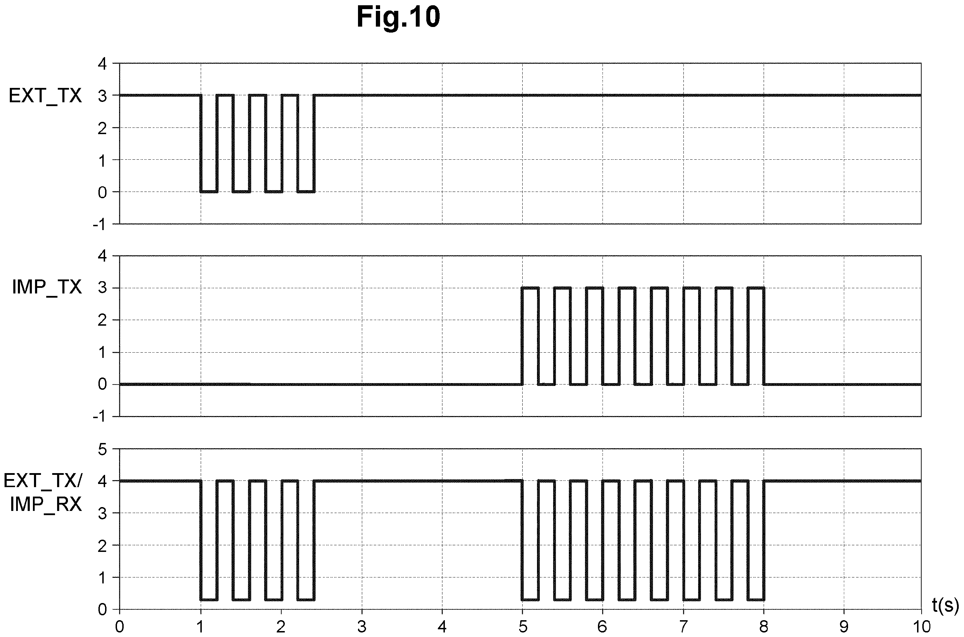

La Figure 10 illustre un exemple de variations du signal de commande EXT_TX, sous forme d'impulsions de tension modulées en amplitude "tout-ou-rien" (OOK), et le signal résultant IMP_RX qui est détecté par le circuit démodulateur 110 de la capsule 10, ce signal reflétant la tension présente sur le couplage 78, 80 entre l'environnement extérieur 48 et la capsule 10.

Du côté de la capsule, le circuit TX/RX 102 comprend un commutateur interne 112 piloté par un signal de commande IMP_TX. Lorsqu'il se ferme, le commutateur 112 court-circuite une résistance 114 de forte valeur montée en parallèle sur la batterie interne 44 et la diode 106, ce qui a pour effet de modifier l'impédance de charge de la capsule 10 vue depuis l'extérieur entre i) une valeur élevée (valeur de l'impédance d'entrée de l'implant entre les bornes AUX et RING, interrupteur 112 ouvert) correspondant à un signal logique haut et ii) une valeur brièvement abaissée (interrupteur 112 fermé) correspondant à un signal logique bas. Ces variations d'impédance sont détectées depuis l'extérieur par le circuit démodulateur 116 qui délivre un signal EXT_RX, ce signal reflétant la tension présente sur le couplage 78, 80 entre l'environnement extérieur 48 et la capsule 10.

Comme illustré sur le chronogramme du bas de la Figure 10, on peut ainsi échanger des signaux sur le conducteur 78, de l'extérieur vers la capsule (les quatre premières impulsions du chronogramme, produites par des coupures de l'alimentation de recharge) et, en réponse, de la capsule vers l'extérieur (les huit impulsions consécutives, produites par la modulation de l'impédance de charge). Les informations échangées pendant le transport et le stockage de la capsule, avant de rendre celle-ci pleinement fonctionnelle, peuvent être très diverses, par exemple :
- de l'extérieur vers la capsule (signaux EXT_TX) : ajustement de paramètres de l'implant avant implantation en fonction de l'état clinique du patient, et commande du basculement de la configuration de transport et de stockage vers la configuration pleinement fonctionnelle ; et
- de la capsule vers l'extérieur (signaux IMP_TX) : tension de la batterie tampon 44, paramètres internes de l'implant, statut de l'implant.

## Revendications

1. Un implant cardiaque autonome notamment de type capsule leadless (10), comprenant :
- un corps tubulaire (12) ;
- des électrodes de surface (70, 72) portées par le corps tubulaire (12) ;
- un module de récupération d'énergie (40) apte à convertir en énergie électrique des sollicitations externes appliquées à l'implant, comprenant 'un ensemble inertiel pendulaire comportant un élément élastiquement déformable (24) couplé à une masse inertielle (26) ;
- une batterie rechargeable (44) apte à être rechargée par le module de récupération d'énergie (40), la batterie étant préalablement chargée à un niveau de charge initial ; et
- un circuit d'interfaçage (86), apte à coupler sélectivement les électrodes de surface (70, 72) à la batterie rechargeable (44),
implant **caractérisé en ce que** le circuit d'interfaçage (86) comprend un circuit de commutation (88-94 ; 88-100) apte à opérer une commutation entre :
• une configuration de transport et de stockage où, avant implantation de l'implant, les électrodes de surface (70, 72) sont reliées (66, 68) à une liaison (78, 80) de couplage à une source externe (82) formant réserve d'énergie électrique, la source externe (82) étant matériellement séparée de l'implant (10),
le circuit d'interfaçage (86) étant adapté pour, dans cette configuration de transport et de stockage, recevoir de la source externe (82) une énergie d'alimentation et délivrer cette énergie d'alimentation à la batterie rechargeable (44), et
• une configuration fonctionnelle où les électrodes de surface (70, 72) sont découplées de la source externe (82) après que l'implant a été implanté,
**en ce que** l'une au moins (72) des électrodes de surface est une électrode auxiliaire (AUX) qui n'est pas une électrode de détection/stimulation de potentiels cardiaques,
et **en ce que** :
• dans la configuration de transport et de stockage, le circuit d'interfaçage (86) est apte à coupler l'électrode auxiliaire à la batterie rechargeable (44) de l'implant, et
• dans la configuration fonctionnelle, le circuit d'interfaçage (86) est apte à découpler l'électrode auxiliaire d'avec la batterie rechargeable (44) de l'implant et apte à mettre l'électrode auxiliaire à un potentiel flottant.

2. L'implant de la revendication 1, dans lequel le circuit de commutation (88-94 ; 88-100) est un circuit apte à opérer une commutation irréversible de la configuration de transport et de stockage vers la configuration fonctionnelle.

3. L'implant de la revendication 2, dans lequel le circuit de commutation (88-94 ; 88-100) comprend au moins un composant (92, 94 ; 92, 94, 98, 100) de type *One-Time Programmable,* OTP.

4. L'implant de la revendication 3, dans lequel le circuit de commutation (88-94 ; 88-100) comprend au moins un commutateur MOS (88, 90 ; 88, 90, 96) dont la grille est commandée par le composant OTP (92, 94 ; 92, 94, 98, 100).

5. L'implant de la revendication 1, dans lequel l'une au moins (32 ; 70) des électrodes de surface est une électrode de détection/stimulation de potentiels cardiaques, et dans lequel :
- dans la configuration de transport et de stockage, le circuit d'interfaçage (86) couple l'électrode de détection/stimulation (32 ; 70) à la batterie rechargeable (44) de l'implant et découple l'électrode de détection/ stimulation (32 ; 70) d'avec un circuit de détection/stimulation (28, 38) de l'implant, et
- dans la configuration fonctionnelle, le circuit d'interfaçage (86) couple l'électrode de détection/stimulation (32 ; 70) au circuit de détection/ stimulation (28, 38) de l'implant et découple l'électrode de détection/ stimulation (32 ; 70) d'avec la batterie rechargeable (44) de l'implant.

6. L'implant de la revendication 5, dans lequel l'électrode de détection/stimulation (32 ; 70) est une électrode annulaire (RING) de l'implant.

7. L'implant de la revendication 1, dans lequel l'implant comprend en outre :
- un circuit émetteur de données (102) apte, dans la configuration de transport et de stockage, à délivrer via les électrodes de surface (70, 72) des signaux de communication (IMP_TX) sur la liaison (78, 80) de couplage à la source externe (82).

8. L'implant de la revendication 7, dans lequel les signaux de communication (IMP_TX) sont des signaux modulés OOK et le circuit émetteur de données (102) comprend un circuit (112, 114) apte à moduler l'impédance de charge de l'implant vue depuis la liaison de couplage à la source externe (82).

9. L'implant de la revendication 7, dans lequel les signaux de communication (IMP_TX) délivrés par le circuit émetteur transmettent des données du groupe comprenant : tension de la batterie rechargeable (44) ; paramètres internes de l'implant ; et statut de l'implant.

10. L'implant de la revendication 1, dans lequel l'implant comprend en outre :
- un circuit récepteur de données (102) apte, dans la configuration de transport et de stockage, à recevoir via les électrodes de surface (70, 72) des signaux de communication (EXT_TX) transmis sur la liaison (78, 80) de couplage à la source externe (82).

11. L'implant de la revendication 10, dans lequel les signaux de communication (EXT_TX) sont des signaux modulés OOK par des coupures impulsionnelles de la liaison de couplage, et le circuit récepteur de données (102) comprend un circuit de démodulation (110) détectant des coupures impulsionnelles de l'énergie d'alimentation reçue de la source externe (82).

12. L'implant de la revendication 10, dans lequel les signaux de communication (EXT_TX) reçus par le circuit récepteur transmettent des données du groupe comprenant : modification de paramètres internes de l'implant ; et commande de la commutation de la configuration de transport et de stockage vers la configuration fonctionnelle.

## Patentansprüche

1. Autonomes Herzimplantat insbesondere vom Typ drahtlose Kapsel (10), umfassend:
- einen rohrförmigen Körper (12);
- Oberflächenelektroden (70, 72), die von dem rohrförmigen Körper (12) getragen werden; ein Energierückgewinnungsmodul (40), das imstande ist, äußere Belastungen, die auf das Implantat aufgebracht werden, in elektrische Energie umzuwandeln, umfassend eine pendelartige inertiale Anordnung mit einem elastisch verformbaren Element (24), das mit einer trägen Masse (26) gekoppelt ist;
- eine wiederaufladbare Batterie (44), die imstande ist, durch das Energierückgewinnungsmodul (40) wiederaufgeladen zu werden, wobei die Batterie zuvor auf einen anfänglichen Ladezustand geladen wird; und
- eine Schnittstellenschaltung (86), die imstande ist, selektiv die Oberflächenelektroden (70, 72) mit der wiederaufladbaren Batterie (44) zu koppeln, wobei das Implantat **dadurch gekennzeichnet ist, dass** die Schnittstellenschaltung (86) eine Umschaltungsschaltung (88-94; 88-100) umfasst, die imstande ist, eine Umschaltung durchzuführen zwischen:
einer Transport- und Speicherkonfiguration, wo, vor Einsetzen des Implantats, die Oberflächenelektroden (70, 72) mit einer Kopplungsverbindung (78, 80) mit einer externen Quelle (82) verbunden (66, 68) sind, die eine elektrische Energiereserve bildet, wobei die externe Quelle (82) von dem Implantat (10) materiell getrennt ist,
wobei die Schnittstellenschaltung (86) eingerichtet ist, um, in dieser Transport- und
Speicherkonfiguration, von der externen Quelle (82) eine Versorgungsenergie aufzunehmen und diese Versorgungsenergie an die wiederaufladbare Batterie (44) abzugeben, und
einer Betriebskonfiguration, wo die Oberflächenelektroden (70, 72) von der externen Quelle (82) entkoppelt sind, nachdem das Implantat eingesetzt wurde,
**dass** wenigstens eine (72) der Oberflächenelektroden eine Hilfselektrode (AUX) ist, die keine Elektrode zur Detektion/Stimulation von Herzpotentialen ist,
**und dass:**
in der Transport- und Speicherkonfiguration, die Schnittstellenschaltung (86) imstande ist, die Hilfselektrode mit der wiederaufladbaren Batterie (44) des Implantats zu koppeln, und
in der Betriebskonfiguration, die Schnittstellenschaltung (86) imstande ist, die Hilfselektrode von der wiederaufladbaren Batterie (44) des Implantats zu entkoppeln und die Hilfselektrode potentialfrei zu setzen.

2. Implantat nach Anspruch 1, wobei die Umschaltungsschaltung (88-94; 88-100) eine Schaltung ist, die imstande ist, eine irreversible Umschaltung von der Transport- und Speicherkonfiguration in die Betriebskonfiguration durchzuführen.

3. Implantat nach Anspruch 2, wobei die Umschaltungsschaltung (88-94; 88-100) wenigstens ein Bauelement (92, 94; 92, 94, 98, 100) vom Typ *One Time Programmable* (OTP) umfasst.

4. Implantat nach Anspruch 3, wobei die Umschaltungsschaltung (88-94; 88-100) wenigstens einen MOS-Schalter (88, 90; 88, 90, 96) umfasst, dessen Gate durch das OTP-Bauelement (92, 94; 92, 94, 98, 100) gesteuert wird.

5. Implantat nach Anspruch 1, wobei wenigstens eine (32; 70) der Oberflächenelektroden eine Elektrode zur Detektion/Stimulation von Herzpotentialen ist und wobei:
- in der Transport- und Speicherkonfiguration, die Schnittstellenschaltung (86) die Detektions-/Stimulations-Elektrode (32; 70) mit der wiederaufladbaren Batterie (44) des Implantats koppelt und die Detektions-/Stimulations-Elektrode (32; 70) von einer Detektions-/Stimulations-Schaltung (28, 38) des Implantats entkoppelt, und
- in der Betriebskonfiguration, die Schnittstellenschaltung (86) die Detektions-/Stimulations-Elektrode (32; 70) mit der Detektions-/Stimulations-Schaltung (28, 38) des Implantats koppelt und die Detektions-/Stimulations-Elektrode (32; 70) von der wiederaufladbaren Batterie (44) des Implantats entkoppelt.

6. Implantat nach Anspruch 5, wobei die Detektions-/Stimulations-Elektrode (32; 70) eine Ringelektrode (RING) des Implantats ist.

7. Implantat nach Anspruch 1, wobei das Implantat ferner umfasst:
- eine Datensendeschaltung (102), die imstande ist, in der Transport- und Speicherkonfiguration, über die Oberflächenelektroden (70, 72) Kommunikationssignale (IMP_TX) auf der Kopplungsverbindung (78, 80) der externen Quelle (82)) bereitzustellen.

8. Implantat nach Anspruch 7, wobei die Kommunikationssignale (IMP_TX) modulierte OOK-Signale sind und die Datensendeschaltung (102) eine Schaltung (112, 114) umfasst, die imstande ist, die Ladeimpedanz des Implantats von der Kopplungsverbindung aus zu der externen Quelle (82) hin gesehen zu modulieren.

9. Implantat nach Anspruch 7, wobei die von der Sendeschaltung bereitgestellten Kommunikationssignale (IMP_TX) Daten der Gruppe übertragen, die umfasst: Spannung der wiederaufladbaren Batterie (44); interne Parameter des Implantats; und Status des Implantats.

10. Implantat nach Anspruch 1, wobei das Implantat ferner umfasst:
- eine Datenempfangsschaltung (102), die imstande ist, in der Transport- und Speicherkonfiguration, über die Oberflächenelektroden (70, 72) Kommunikationssignale (EXT_TX), die auf der Kopplungsverbindung (78, 80) an die externe Quelle (82) übertragen werden, zu empfangen.

11. Implantat nach Anspruch 10, wobei die Kommunikationssignale (EXT_TX) OOK-Signale sind, die durch Impulsunterbrechungen der Kopplungsverbindung moduliert sind, und die Datenempfangsschaltung (102) eine Demodulationsschaltung (110) umfasst, die Impulsunterbrechungen der von der externen Quelle (82) aufgenommenen Versorgungsenergie detektiert.

12. Implantat nach Anspruch 10, wobei die Kommunikationssignale (EXT_TX), die von der Empfangsschaltung empfangen werden, Daten der Gruppe übertragen, die umfasst: Änderung von internen Parametern des Implantats; und Steuerung der Umschaltung von der Transport- und Speicherkonfiguration in die Betriebskonfiguration.

## Claims

1. An autonomous cardiac implant, in particular of the leadless capsule type (10), comprising:
- a tubular body (12);
- surface electrodes (70, 72) carried by the tubular body (12);
- an energy harvesting module (40) adapted to convert external stresses applied to the implant into electrical energy, comprising an inertial pendular unit including an elastically deformable element (24) coupled to an inertial mass (26);
- a rechargeable battery (44) adapted to be charged by the energy harvesting module (40), the battery being previously charged to an initial charge level; and
- an interface circuit (86) adapted to selectively couple the surface electrodes (70, 72) to the rechargeable battery(44),
said implant being **characterized in that** the interface circuit (86) comprises a switching circuit (88-94; 88-100) adapted to operate a switching between:
• a transport and storage configuration in which, before implantation of the implant, the surface electrodes (70, 72) are connected (66, 68) to a link (78, 80) coupling to an external source (82) forming an electrical energy reserve, the external source (82) being physically separated from the implant (10), the interface circuit (86) being adapted, in said transport and storage configuration, to receive a power supply from the external source (82) and to provide said power supply to the rechargeable battery (44), and
• a functional configuration, in which the surface electrodes (70, 72) are decoupled from the external source (82) after the implant has been implanted,
**in that** at least one (72) of the surface electrodes is an auxiliary electrode (AUX) that is not an electrode for pacing/detecting cardiac potentials,
and **in that:**
• in the transport and storage configuration, the interface circuit (86) is adapted to couple the auxiliary electrode to the rechargeable battery (44) of the implant, and
• in the functional configuration, the interface circuit (86) is adapted to decouple the auxiliary electrode from the rechargeable battery (44) of the implant, and is adapted to put the auxiliary electrode to a floating potential.

2. The implant of claim 1, wherein the switching circuit (88-94; 88-100) is a circuit adapted to operate an irreversible switching from the transport and storage configuration to the functional configuration.

3. The implant of claim 2, wherein the switching circuit (88-94; 88-100) comprises at least one component (92, 94; 92, 94, 98, 100) of the One-Time Programmable, OTP, type.

4. The implant of claim 3, wherein the switching circuit (88-94; 88-100) comprises at least one MOS switch (88, 90; 88, 90, 96) the gate of which is controlled by the OTP component (92, 94; 92, 94, 98, 100).

5. The implant of claim 1, wherein at least one (32; 70) of the surface electrodes is an electrode for pacing/detecting cardiac potentials, and wherein:
- in the transport and storage configuration, the interface circuit (86) couples the pacing/detecting electrode (32; 70) to the rechargeable battery (44) of the implant, and decouples the pacing/detecting electrode (32; 70) from a pacing/detecting circuit (28, 38) of the implant, and
- in the functional configuration, the interface circuit (86) couples the pacing/detecting electrode (32; 70) to the pacing/detecting circuit (28, 38) of the implant, and decouples the pacing/detecting electrode (32; 70) from the rechargeable battery (44) of the implant.

6. The implant of claim 5, wherein the pacing/detecting electrode (32; 70) is a ring electrode (RING) of the implant.

7. The implant of claim 1, wherein the implant further comprises:
- a data transmitter circuit (102) adapted, in the transport and storage configuration, to send via the surface electrodes (70, 72) communication signals (IMP_TX) on the coupling link (78, 80) to the external source (82).

8. The implant of claim 7, wherein the communication signals (IMP_TX) are OOK-modulated signals, and the data transmitter circuit (102) comprises a circuit (112, 114) adapted to modulate the load impedance of the implant as viewed from the coupling link to the external source (82).

9. The implant of claim 7, wherein the communication signals (IMP_TX) output by the transmitter circuit convey data from the group including: voltage of the rechargeable battery (44); internal parameters of the implant; and status of the implant.

10. The implant of claim 1, wherein the implant further comprises:
- a data receiver circuit (102) adapted, in the transport and storage configuration, to receive via the surface electrodes (70, 72) communication signals (EXT_TX) transmitted on the coupling link (78, 80) to the external source (82).

11. The implant of claim 10, wherein the communication signals (EXT_TX) are signals OOK-modulated by pulse cutoffs of the coupling link, and the data receiver circuit (102) comprises a demodulation circuit (110) detecting pulse cutoffs of the power supply received from the external source (82).

12. The implant of claim 10, wherein the communication signals (EXT_TX) received by the receiver circuit transmit data from the group including: changes to internal parameters of the implant; and control of the switching from the transport and storage configuration to the functional configuration.
